# EUROPEAN PATENT SPECIFICATION

(11) **EP 2 191 779 B1**
(45) Date of publication and mention of the grant of the patent: **04.04.2012**
(21) Application number: 09014649.9
(22) Date of filing: 24.11.2009
(51) Int. Cl.: A61B 17/70

(54) **Dynamic stabilization system components including readily visualized polymeric compositions**
Dynamische Stabilisierungssystemkomponenten mit fertig visualisierten Polymerzusammensetzungen
Composants de système de stabilisation dynamique incluant des compositions polymériques visualisées facilement

(30) Priority: 01.12.2008 US 325727
(43) Date of publication of application: 02.06.2010
(73) Proprietor: Zimmer Spine, Inc., Minneapolis, MN 55439-2027 (US)
(72) Inventor: Zhang, Kai, Woodbury, Minnesota 55125 (US)
(74) Representative: Manitz, Finsterwald & Partner GbR

(56) References cited:
- WO-A1-2008/115622
- US-A1- 2005 033 295

## Description

### Technical Field

The disclosure is directed to a vertebral stabilization system. More particularly, the disclosure is directed to a dynamic stabilization system including one or more components including a readily visualized polymeric composition and a method of use thereof.

### Background

The spinal column of a patient includes a plurality of vertebrae linked to one another by facet joints and an intervertebral disc located between adjacent vertebrae. The facet joints and intervertebral disc allow one vertebra to move relative to an adjacent vertebra, providing the spinal column a range of motion. Diseased, degenerated, damaged, or otherwise impaired facet joints and/or intervertebral discs may cause the patient to experience pain or discomfort and/or loss of motion, thus prompting surgery to alleviate the pain and/or restore motion of the spinal column.

Accordingly, there is an ongoing need to provide alternative devices, assemblies, systems and/or methods that can function to alleviate pain or discomfort, provide stability, such as dynamic stability, and/or restore a range of motion to a spinal segment of a spinal column.

Document WO 2008 / 115622 A1 discloses a vertebral stabilization system according to the preamble of claim 1.

It may be desirable that such apparatus or components thereof, exhibit a degree of radiopacity in order that the apparatus may be visualized with a fluoroscopy device or other visualization device during a medical procedure and/or during a post-operative reevaluation.

### Summary

A system with these properties is provided by a vertebral stablilization system with the features recited by claim 1.

The invention relates to a vertebral stabilization system comprising a flexible elongate member, such as a cord, and a second elongate member, such as a spacer, at least partially surrounding the flexible elongate member (e.g., cord). At least one of the first elongate member and the second elongate member (e.g., the cord and/or the spacer) may comprise the polymerized residue of at least one monomer selected from the group consisting of halogen substituted (meth)acrylates, covalent salts of Group II elements other than beryllium, and chelates of Group II elements other than beryllium. In some instances, at least one of the cord and the spacer may be provided with markings suitable for use in a radiographic comparison method as described herein, said markings comprising the polymerized residue of at least one monomer selected from the group consisting of halogen substituted (meth)acrylates, covalent salts of Group II elements other than beryllium, and chelates of Group II elements other than beryllium. When assembled between first and second vertebral anchors, the cord may be in tension and the spacer may be in compression.

The invention is also directed to a method of improving the selective radiopacity of polymeric components of vertebral stabilization systems by providing a vertebral stabilization system comprising a flexible elongate member, such as a cord, extendable from a first vertebral anchor to a second vertebral anchor, and a second elongate member, such as a spacer, sized to surround a portion of the first elongate member (e.g., cord) between the first vertebral anchor and the second vertebral anchor. The method further comprises providing at least one monomer selected from the group consisting of halogen substituted (meth)acrylates, covalent salts of Group II elements other than beryllium, and chelates of Group II elements other than beryllium and applying the monomer to at least a portion of one of the first elongate member and the second elongate member (e.g. at least one of the cord and the spacer) and polymerizing the monomer. The vertebral stabilization system may be assembled by placing the spacer around the cord between a first vertebral anchor and a second vertebral anchor, securing the cord to the first vertebral anchor, and securing the cord to the second vertebral anchor such that the cord is in tension and the spacer is in compression. It will be appreciated that steps such as the monomer application and polymerization steps may be exchanged with the steps associated with assembling the vertebral stabilization system at the convenience of the operator.

A method of monitoring the stability of a vertebral stabilization system by providing a vertebral stabilization system is described above, which is not part of the present invention, as obtaining a first radiographic image of the vertebral stabilization system, waiting an interval of time, obtaining a second radiographic image of the vertebral stabilization system, comparing the first radiographic image to the second radiographic image, and noting differences between the first radiographic image and the second radiographic image.

The above summary of some example embodiments is not intended to describe each disclosed embodiment or every implementation of the invention.

### Brief Description of the Drawings

The invention may be more completely understood in consideration of the following detailed description of various embodiments in connection with the accompanying drawings, in which:
FIGURE 1 is a perspective view of an exemplary vertebral stabilization system;
FIGURES 2A-D are schematically illustrative of pattern-wise application of monomer(s);
FIGURES 3A-D are schematically illustrative of pattern-wise application of monomer(s); and
FIGURES 4A-B are schematically illustrative of first and second radiographic images.

While the invention is amenable to various modifications and alternative forms, specifics thereof have been shown by way of example in the drawings and will be described in detail. It should be understood, however, that the intention is not to limit aspects of the invention to the particular embodiments described.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

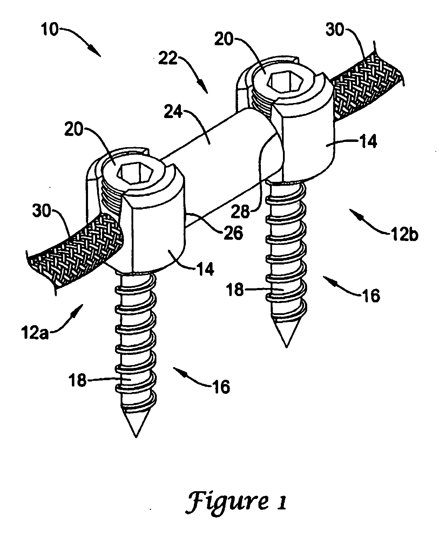

Referring now to FIG. 1, there is shown a vertebral fixation system 10 for stabilizing a portion of a spinal column, such as one or more spinal segments of a spinal column. As used herein, a spinal segment is intended to refer to two or more vertebrae, the intervertebral disc(s) between the vertebrae and other anatomical elements between the vertebrae. For example, a spinal segment may include first and second adjacent vertebrae and the intervertebral disc located between the first and second vertebrae. The spinal stabilization system 10 may provide dynamic stabilization to a spinal segment, preserving and/or allowing for a range of motion of the spinal segment.

The vertebral stabilization system 10 may be used to treat discogenic low back pain, degenerative spinal stenosis, disc herniations, facet syndrome, posterior element instability, adjacent level syndrome associated with spinal fusion, and/or other maladies associated with the spinal column.

The vertebral stabilization system 10 may include one or more or a plurality of vertebral anchors or fasteners 12. Although the vertebral anchors 12 are depicted as threaded vertebral fasteners (e.g., pedicle screws, bone screws), in some embodiments the vertebral anchors 12 may be vertebral books (e.g., laminar hooks) or other types of fastening members for attachment to a bony structure such as a vertebra of the spinal column. Each of the vertebral anchors 12 may be configured to be secured to a vertebra of a spinal column. For instance, the first vertebral anchor 12a may be secured to a first vertebra and the second vertebral anchor 12b may be secured to a second vertebra. Additional vertebral anchors 12 may be present in instances in which the vertebral stabilization system 10 spans three or more vertebra of the spinal column.

The vertebral anchor 12 may include a head portion 14 and a bone engagement portion 16 extending from the head portion 14. In some embodiments, the bone engagement portion 16 may be a shaft portion 18 of the vertebral anchor 12 extending from the head portion 14 along a longitudinal axis of the vertebral anchor 12. In some embodiments, the vertebral anchor 12 may be a monoaxial screw, and in other embodiments the vertebral anchor 12 may be a polyaxial screw. In some embodiments, the shaft portion 18 may be configured to be installed into a bony region of a vertebra of the spinal column. For example, the shaft portion 18 may be installed into a pedicle of a vertebra, or other region of a vertebra. In some embodiments, the shaft portion 18 may be a threaded region having helical threads configured to be screwed into a pedicle of a vertebra, or other bony region of a vertebra.

The vertebral anchor 12 may include a securing element, such as a threaded fastener 20 (e.g., a set screw, cap) configured to engage the head portion 14 to secure a portion of a connecting member 22 to the vertebral anchor 12. For example, the threaded fastener 20 may include threads which mate with threads formed in the head portion 14.

The vertebral stabilization system 10 may also include one or more, or a plurality of connecting members 22 extending between vertebral anchors 12 of the vertebral stabilization system 10. As an illustrative example, the vertebral stabilization system 10 shown in FIG. 1 includes a connecting member 22 extending between the first vertebral anchor 12a and the second vertebral anchor 12b.

The connecting member 22 may be constructed of a plurality of components in some instances. For instance, the connector 22 may include a spacer 24, and a cord 30 extending through the spacer 24, as well as other components if desired.

In some embodiments, the spacer 24 may be an annular spacer having a lumen (not shown) extending from a first end 26 to a second end 28 of the spacer 24. For example, in some embodiments the spacer 24 may be a cylindrical member having a lumen extending therethrough. In other embodiments, the spacer 24 may be molded, extruded, or otherwise formed over and/or around the cord 30. The spacer 24 may be positioned between the head portion 14 of the first vertebral anchor 12a and the head portion 14 of the second vertebral anchor 12b. For instance, when installed between the first and second vertebral anchors 12a, 12b, the first end 26 of the spacer 24 may face, abut or otherwise contact a side surface of the head portion 14 of the first vertebral anchor 12a, and the second end 28 of the spacer 24 may face, abut or otherwise contact a side surface of the head portion 14 of the second vertebral anchor 12b.

The cord 30 may extend from the head portion 14 of the first vertebral anchor 12a to the head portion 14 of the second vertebral anchor 12b. In some embodiments, the cord 30 may extend into and/or extend through a channel, such as a U-shaped channel, extending through the head portion 14 of the first vertebral anchor 12a, and the cord 30 may extend into and/or extend through a channel, such as a U-shaped channel, extending through the head portion 14 of the second vertebral anchor 12b. In some embodiments, the threaded fastener 20 of the first vertebral anchor 12a may be tightened directly onto the cord 30 to retain the cord 30 in the channel of the head portion 14 of the first vertebral anchor 12a, and/or the threaded fastener 20 of the second vertebral anchor 12b may be tightened directly onto the cord 30 to retain the cord in the channel of the head portion 14 of the second vertebral anchor 12b. In other embodiments, the cord 30 may extend into, extend through, and/or be secured to another component which spaces the cord 30 from direct contact with the channel of the vertebral anchor 12a, 12b. For example, the cord 30 may extend into, extend through, and/or be secured to a spindle, spool, sleeve, coupler, or other component, which in turn is secured in the channel of the head portion of the vertebral anchor 12a, 12b with the threaded fastener 20 or other securing fastener. It is noted that during a medical procedure the portions of the cord 30 which are shown extending from the channels of the vertebral anchors 12a, 12b may be trimmed as desired to reduce and/or eliminate the portion of the cord 30 extending from the vertebral anchors 12a, 12b.

When implanted in a patient, the cord 30 of the vertebral stabilization system 10 may limit the range of flexion of the spinal segment, whereas the spacer 24 may limit the range of extension of the spinal segment. For instance, the cord 30 may be placed in tension and the spacer 24 may be placed in compression between the vertebral anchors 12a, 12b.

At least one of the cord 30 and the spacer 24 may comprise a polymerized monomer which may be readily visualized when the monomer or monomers are selected from halogen substituted (meth)acrylates, covalent salts of Group II elements other than beryllium, and chelates of Group II elements other than beryllium. For instance, in some embodiments the cord 30 and/or the spacer 24 may comprise an iodine-containing monomer, such as an iodine-containing acrylate, or other halogen-containing monomer. In some instances, the halogen-containing monomer, or other radiolucent monomer, may be in a coating applied to the surface of the bulk material of the cord 30 and/or the spacer 24. In other instances, the halogen-containing monomer, or other radiolucent monomer, may be polymerized in the bulk material of the cord 30 and/or the spacer 24. In some embodiments, the bulk material of the cord 30 and/or the spacer 24 may include polyurethane, polycarbonate urethane, polyethylene, polyethylene terephthalate, polybutylene terephthalate, tepolymethyl methacrylate, polyaryl ether ketone, blends or copolymers with at least one of the above polymers as a component.

In some embodiments, the cord 30 and/or the spacer 24 may include a marking comprising the at least one polymerized monomer selected from the group consisting of halogen substituted (meth)acrylates, covalent salts of Group II elements other than beryllium, and chelates of Group II elements other than beryllium.

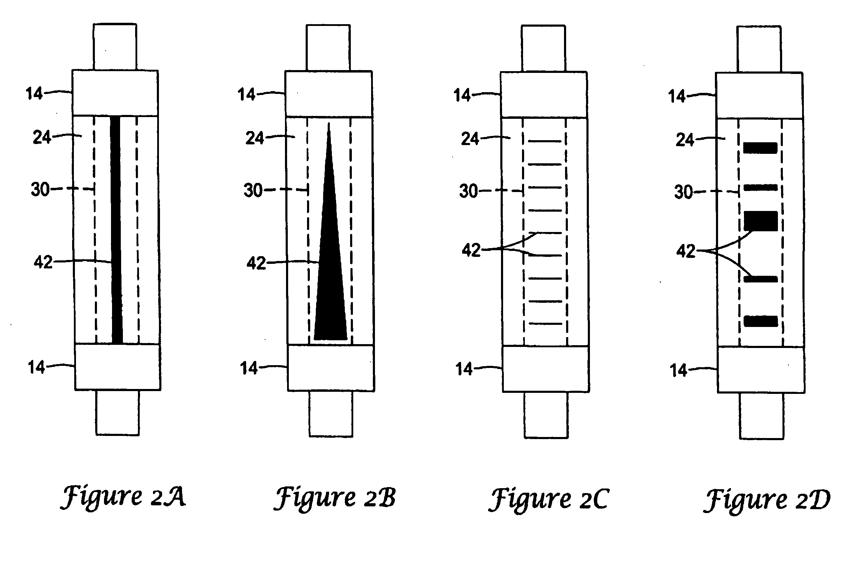

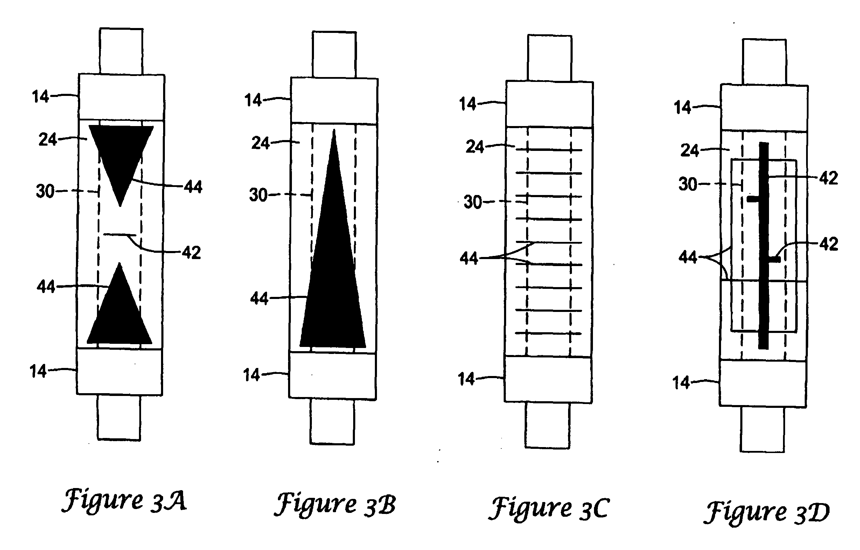

FIGURES 2A-2D and 3A-3D provide schematic illustrative examples of pattern-wise deposited and polymerized monomers which may be readily visualized when the monomer or monomers are selected from halogen substituted (meth)acrylates, covalent salts of Group II elements other than beryllium, and chelates of Group II elements other than beryllium. In these FIGURES 2A-2D, the monomer has been applied to the cord 30 and polymerized prior to assembly of the vertebral stabilization system. FIG. 2A represents the marking in the form of one or more simple uniform width longitudinal stripes 42 applied to the cord 30 which could be used to determine if the cord 30 had been twisted during tensioning and clamping. Figure 2B depicts a longitudinal tapered line which clearly indicates orientation of the cord 30 as well as any applied torque. FIGURE 2C provides a uniformly spaced transverse bar pattern which may be applied to the cord 30 to indicate the spacing between the first and second head portions 14 of vertebral anchors 12. The asymmetric pattern of FIGURE 2D combines an orientation indication with transverse markings which may be used to judge the distance between the first and second vertebral anchors 12.

In FIGURES 3A-3D, the monomer has been applied to spacer 24 in a pattern-wise manner and polymerized. In FIG. 3A, the resulting markings 44 cooperate with mark 42 on the cord 30 to indicate the relative positioning of the cord 30 and spacer 24. As in FIG. 2B, the asymmetry in the markings applied to the spacer 24 may confirm the orientation of the vertebral stabilization system 10. Although the mark 42 would generally be placed and polymerized on cord 30 prior to assembly of the stabilization system 10, it may be desirable to apply the monomer(s) which will result in marks 44 to spacer 24 after the stabilization system 10 is in place to ensure that the markings are aligned in the desired relative relationship. The markings may be applied by any of the commonly employed methods, for example by rubber stamp. In such embodiments, the monomers may be, for example, photopolymerized *in situ.*

FIG. 3B illustrates an asymmetric mark 44 applied to spacer 24, said mark indicating the orientation of the spacer 24. The uniformly distributed marks 44 applied to spacer 24 of FIG. 3C may be used to judge the degree of compression of the spacer 24 following assembly of the vertebral stabilization system 10. In FIG. 3D, a combination of markings 42 applied to the cord 30 and marks 44 applied to spacer 24 may be used to judge the relative positions of the cord 30 and spacer 24 as well as any torques introduced into the system.

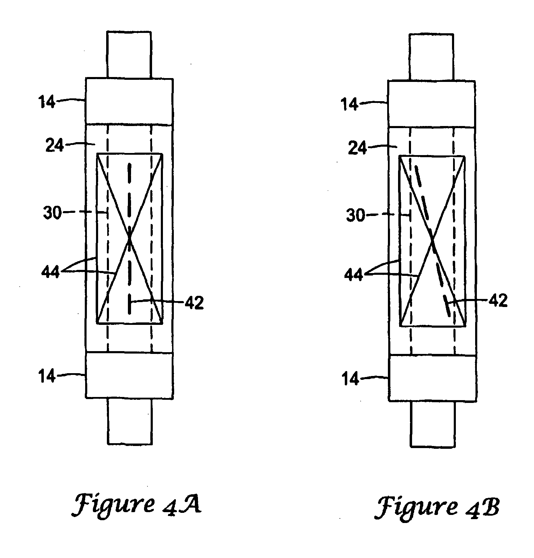

In FIG. 4A, the relative positions of spacer 24 and cord 30, as indicated by markings 42 and 44, have been captured as they might appear in a first reference radiographic image. At a later time, a second radiographic image Fig. 4B has been captured. A comparison of the two images indicates that the mark 42 on cord 30 has shifted relative to the marks 44 on spacer 24 during the interval between the two images, perhaps as the result of slippage between cord 30 and one of the vertebral anchors 12. In this manner, the vertebral stabilization system 10 may be monitored over its useful life by periodically acquiring a new radiographic image for comparison. The detection of a change in alignment may indicate that the vertebral stabilization system 10 should be replaced, reconfigured, or otherwise adjusted. The evaluation may be performed without the need to expose the vertebral stabilization system 10 for direct observation.

In some embodiments, the monomer is applied in a pattern-wise manner. The pattern may be applied to either or both of the cord 30 and the spacer 24. The pattern or patterns may comprise uniform elements uniformly spaced or may comprise a combination of uniform or non-uniform elements as well as uniformly or non-uniformly spaced elements. In certain embodiments, patterned elements applied to the cord 24 will have a determined, or at least determinable, spatial relationship to a marking or markings on the spacer 24. In some embodiments, the markings may include generally axially elongated elements such as one or more stripes, triangles, ellipses, broken line segments, a sequence of dots, or the like. In other embodiments, the markings may be oriented generally transversely relative to a long axis of the device. In yet other embodiments, the markings may include a combination of axial and transversely oriented elements. In certain embodiments, the markings applied to the cord 30 will resemble the markings applied to the spacer 24 while in other embodiments the markings will differ in at least one of form and orientation.

In selecting the monomers to be used in marking the cord 30 and spacer 24, it is desirable that the resulting polymer be readily visualized by conventional means both before and after the vertebral stabilization system 10 has been installed. In some embodiments, it is desirable to select monomers which contribute high electron density to the resulting polymer while maintaining biocompatibility and avoiding components which may release toxic or otherwise undesirable species. It has been found that polymers derived from halogenated monomers, particularly halogenated acrylic and methacrylic monomers, often referred to collectively as (meth)acrylates, are well suited for this purpose. Although various halogenated species may be useful in this regard, triiodo aromatic derivatives of (meth)acrylic may be employed, alone or in combination with other monomers, to provide a polymer which has relatively high electron density and low toxicity. Alternatively monomers comprising covalent salts or chelates of metals having high electron density may also be employed. Of the metals, it is believed that multivalent metals are particularly well suited for this purpose in that they tend to form polydentate salts and/or chelation complexes which are resistant to undesirable release of the metal or metal ion. Of the metals, the higher atomic number Group **II** elements have been found to provide a good balance between high electron density and stability of the salt or complex. While halogenated (meth)acrylic monomers may readily be polymerized by addition polymerization, it will be appreciated that monomers which include covalent salts or chelates of Group II elements may be selected which polymerize by addition or condensation polymerization mechanisms.

## Claims

1. A vertebral stabilization system comprising:
a flexible cord (30); and
a spacer (24); surrounding a portion of the cord (30);
**characterised in that**:
at least one of the cord (30) and the spacer (24) comprises at least one polymerized monomer selected from the group consisting of halogen substituted (meth)acrylates, covalent salts of Group II elements other than beryllium, and chelates of Group II elements other than beryllium.

2. The vertebral stabilization system of claim 1, wherein at least one of the cord (30) and the spacer (24) incudes a marking (42,44) comprising the at least one polymerized monomer selected from the group consisting of halogen substituted (meth)acrylates, covalent salts of Group II elements other than beryllium, and chelates of Group II elements other than beryllium.

3. The vertebral stabilization system of claim 2, wherein the marking includes a plurality of non-uniform stripes (42,44).

4. The vertebral stabilization system of claim 3, wherein the plurality of non-uniform stripes (42,44) extend generally parallel to a longitudinal axis of the vertebral stabilization system.

5. The vertebral stabilization system of claim 3, wherein the plurality of non-uniform stripes (42,44) are transverse to a longitudinal axis of the vertebral stabilization system.

6. The vertebral stabilization system of claim 5, wherein the plurality of non-uniform stripes (42,44) are uniformly spaced, or wherein the plurality of non-uniform stripes (42,44) are non-uniformly spaced.

7. The vertebral stabilization system of claim 2, wherein the marking includes a plurality of uniform stripes (42,44).

8. The vertebral stabilization system of claim **7,** wherein the plurality of uniform stripes (42,44) extend generally parallel to a longitudinal axis of the vertebral stabilization system.

9. The vertebral stabilization system of claim 7, wherein the plurality of uniform stripes (42,44) are transverse to a longitudinal axis of the vertebral stabilization system.

10. The vertebral stabilization system of claim 9, wherein the plurality of uniform stripes (42,44) are uniformly spaced, or wherein the plurality of uniform stripes (42,44) are non-uniformly spaced.

11. A method of improving the selective visualization of polymeric components of a vertebral stabilization system, the method comprising:
providing a vertebral stabilization including a cord (30) and a spacer (24) sized to surround a portion of the cord (30);
providing at least one monomer selected from the group consisting of halogen substituted (meth)acrylates, covalent salts of Group **II** elements other than beryllium, and chelates of Group II elements other than beryllium;
applying the monomer to at least a portion of one of the cord (30) and the spacer (24); and
polymerizing the monomer.

12. The method of claim 11, wherein the step of applying the monomer comprises applying the monomer in a pattern-wise manner.

13. The method of claim 12, wherein applying the monomer in a pattern-wise manner includes a applying the monomer in a plurality of uniformly oriented marks (42,44); or wherein applying the monomer in a pattern-wise manner includes a applying the monomer in a plurality of non-uniformly oriented marks (42,44).

14. The method of claim 12, wherein the pattern-wise manner of monomer application includes one or more non-uniform stripes (42,44), or wherein the pattern-wise manner of monomer application includes one or more uniform stripes (42,44).

15. The method of claim 11, wherein the monomer is at least partially polymerized prior to the applying step.

## Patentansprüche

1. Wirbelstabilisierungssystem, umfassend:
einen flexiblen Cord (30); und
einen Abstandhalter (24), der einen Abschnitt des Cords (30) umgibt;
**dadurch gekennzeichnet, dass**:
der Cord (30) und/oder der Abstandhalter (24) zumindest ein polymerisiertes Monomer umfassen, das aus der Gruppe gewählt ist, die aus halogensubstituierten (Meth)Acrylaten, kovalenten Salzen von Elementen der Gruppe II mit Ausnahme von Beryllium und Chelaten von Elementen der Gruppe II mit Ausnahme von Beryllium besteht.

2. Wirbelstabilisierungssystem nach Anspruch 1,
wobei der Cord (30) und/oder der Abstandhalter (24) eine Markierung (42, 44) umfassen, die das zumindest eine polymerisierte Monomer umfasst, das aus der Gruppe gewählt ist, die aus halogensubstituierten (Meth)Acrylaten, kovalenten Salzen von Elementen der Gruppe II mit Ausnahme von Beryllium und Chelaten von Elementen der Gruppe II mit Ausnahme von Beryllium besteht.

3. Wirbelstabilisierungssystem nach Anspruch 2,
wobei die Markierung eine Mehrzahl ungleichförmiger Streifen (42, 44) aufweist.

4. Wirbelstabilisierungssystem nach Anspruch 3,
wobei sich die Mehrzahl ungleichförmiger Streifen (42, 44) allgemein parallel zu einer Längsachse des Wirbelstabilisierungssystems erstreckt.

5. Wirbelstabilisierungssystem nach Anspruch 3,
wobei die Mehrzahl ungleichförmiger Streifen (42, 44) quer zu einer Längsachse des Wirbelstabilisierungssystems verläuft.

6. Wirbelstabilisierungssystem nach Anspruch 5,
wobei die Mehrzahl ungleichförmiger Streifen (42, 44) gleichförmig beabstandet sind oder wobei die Mehrzahl ungleichförmiger Streifen (42, 44) ungleichförmig beabstandet sind.

7. Wirbelstabilisierungssystem nach Anspruch 2,
wobei die Markierung eine Mehrzahl gleichförmiger Streifen (42 ,44) aufweist.

8. Wirbelstabilisierungssystem nach Anspruch 7,
wobei sich die Mehrzahl gleichförmiger Streifen (42, 44) allgemein parallel zu einer Längsachse des Wirbelstabilisierungssystems erstreckt.

9. Wirbelstabilisierungssystem nach Anspruch 7,
wobei die Mehrzahl gleichförmiger Streifen (42 ,44) quer zu einer Längsachse des Wirbelstabilisierungssystems verläuft.

10. Wirbelstabilisierungssystem nach Anspruch 9,
wobei die Mehrzahl gleichförmiger Streifen (42, 44) gleichförmig beabstandet sind oder wobei die Mehrzahl gleichförmiger Streifen (42, 44) ungleichförmig beabstandet sind.

11. Verfahren zum Verbessern der selektiven Visualisierung polymerer Komponenten eines Wirbelstabilisierungssystems, wobei das Verfahren umfasst:
Bereitstellen einer Wirbelstabilisierung mit einem Cord (30) und einem Abstandhalter (24), der derart bemessen ist, dass er einen Abschnitt des Cords (30) umgibt;
Bereitstellen zumindest eines Monomers, das aus der Gruppe gewählt ist, die aus halogensubstituierten (Meth)Acrylaten, kovalenten Salzen von Elementen der Gruppe II mit Ausnahme von Beryllium und Chelaten von Elementen der Gruppe II mit Ausnahme von Beryllium besteht;
Aufbringen des Monomers auf zumindest einen Abschnitt des Cords (30) oder des Abstandhalters (40); und
Polymerisieren des Monomers.

12. Verfahren nach Anspruch 11,
wobei der Schritt zum Aufbringen des Monomers ein Aufbringen des Monomers in einer musterartigen Weise umfasst.

13. Verfahren nach Anspruch 12,
wobei das Aufbringen des Monomers in einer musterartigen Weise ein Aufbringen des Monomers in einer Mehrzahl gleichförmig orientierter Markierungen (42, 44) aufweist oder wobei das Aufbringen des Monomers in einer musterartigen Weise ein Aufbringen des Monomers in einer Mehrzahl ungleichförmig orientierter Markierungen (42, 44) aufweist.

14. Verfahren nach Anspruch 12,
wobei die musterartige Weise der Monomeraufbringung ein oder mehrere ungleichförmige Streifen (42 ,44) aufweist oder wobei die musterartige Weise der Monomeraufbringung ein oder mehrere gleichförmige Streifen (42, 44) aufweist.

15. Verfahren nach Anspruch 11,
wobei das Monomer vor dem Aufbringungsschritt zumindest teilweise polymerisiert wird.

## Revendications

1. Système de stabilisation vertébrale, comprenant :
un cordon flexible (30) ; et
un élément d'espacement (24) entourant une portion du cordon (30) ;
**caractérisé en ce que** :
un élément au moins parmi le cordon (30) et l'élément d'espacement (24) comprend au moins un monomère polymérisé choisi parmi le groupe comprenant les (méth)acrylates substitués par un halogène, les sels covalents d'éléments du Groupe II autres que le béryllium, et les chélates des éléments du Groupe II autres que le béryllium.

2. Système de stabilisation vertébrale selon la revendication 1, dans lequel un élément au moins parmi le cordon (30) et l'élément d'espacement (24) inclut une marque (42, 44) comprenant ledit au moins un monomère polymérisé choisi parmi le groupe comprenant les (méth)acrylates substitués par un halogène, les sels covalents d'éléments du Groupe II autres que le béryllium, et les chélates des éléments du Groupe II autres que le béryllium.

3. Système de stabilisation vertébrale selon la revendication 2, dans lequel la marque inclut une pluralité de bandes non-uniformes (42, 44).

4. Système de stabilisation verticale selon la revendication 3, dans lequel la pluralité de bande non-uniformes (42, 44) s'étendent généralement parallèles à un axe longitudinal du système de stabilisation vertébrale.

5. Système de stabilisation vertébrale selon la revendication 3, dans lequel la pluralité de bandes non-uniformes (42, 44) sont transversales à un axe longitudinal du système de stabilisation vertébrale.

6. Système de stabilisation vertébrale selon la revendication 5, dans lequel la pluralité de bande non-uniformes (42, 44) sont espacées uniformément, ou dans lequel la pluralité de bandes non-uniformes (42, 44) sont espacées de manière non uniforme.

7. Système de stabilisation vertébrale selon la revendication 2, dans lequel la marque inclut une pluralité de bandes uniformes (42, 44).

8. Système de stabilisation vertébrale selon la revendication 7, dans lequel la pluralité de bandes uniformes (42, 44) s'étendent généralement parallèles à un axe longitudinal du système de stabilisation vertébrale.

9. Système de stabilisation vertébrale selon la revendication 7, dans lequel la pluralité de bandes uniformes (42, 44) sont transversales à un axe longitudinal du système de stabilisation vertébrale.

10. Système de stabilisation vertébrale selon la revendication 9, dans lequel la pluralité de bandes uniformes (42, 44) sont uniformément espacées, ou dans lequel la pluralité de bandes uniformes (42, 44) sont espacées de manière non-uniforme.

11. Procédé pour améliorer la visualisation sélective de composants polymères d'un système de stabilisation vertébrale, le procédé comprenant :
la fourniture d'un système de stabilisation vertébrale qui inclut un cordon (30) et un élément d'espacement (24) d'une taille propre à entourer une portion du cordon (30) ;
la fourniture d'au moins un monomère choisi parmi le groupe comprenant les (méth)acrylates substitués par un halogène, les sels covalents d'éléments du Groupe II autres que le béryllium, et les chélates des éléments du Groupe II autres que le béryllium. ;
l'application du monomère à au moins une portion d'un élément parmi le cordon (30) et l'élément d'espacement (24) ; et
la polymérisation du monomère.

12. Procédé selon la revendication 11, dans lequel l'étape d'application du monomère comprend l'application du monomère suivant un motif.

13. Procédé selon la revendication 12, dans lequel l'application du monomère suivant un motif inclut une application du monomère dans une pluralité de marques orientées uniformément (42, 44) ; ou dans lequel l'application du monomère suivant un motif inclut une application du monomère dans une pluralité de marques orientées de manière non-uniforme (42, 44).

14. Procédé selon la revendication 12, dans lequel le motif d'application du monomère inclut une ou plusieurs bandes non-uniformes (42, 44), ou dans lequel le motif d'application du monomère inclut une ou plusieurs bandes uniformes (42, 44).

15. Procédé selon la revendication 11, dans lequel le monomère est au moins partiellement polymérisé avant l'étape d'application.
